# EUROPEAN PATENT SPECIFICATION

(11) **EP 2 938 362 B1**
(45) Date of publication and mention of the grant of the patent: **07.12.2016**
(21) Application number: 13834323.1
(22) Date of filing: 17.12.2013
(51) Int. Cl.: A61K 45/06, A61K 31/40, A61K 9/20, A61K 31/155

(54) **DRY GRANULATION PROCESS FOR PRODUCING TABLET COMPOSITIONS OF METFORMIN AND COMPOSITIONS THEREOF**
TROCKENGRANULATIONSVERFAHREN ZUR HERSTELLUNG VON TABLETTENZUSAMMENSETZUNGEN AUS METFORMIN UND ZUSAMMENSETZUNGEN DAVON
PROCÉDÉ DE GRANULATION À SEC POUR LA PRODUCTION DE COMPOSITIONS DE COMPRIMÉS DE METFORMINE ET COMPOSITIONS ASSOCIÉES

(30) Priority: 27.12.2012 TR 201215479
(43) Date of publication of application: 04.11.2015
(73) Proprietor: Zentiva Saglik Ürünleri San. ve Tic. A.S., 34394 Istanbul (TR)
(72) Inventor: ASKAR, Gözde, 39780 Lüleburgaz / Kirklareli (TR); ORAN, Umut, 39780 Lüleburgaz / Kirklareli (TR); BITIK, Naci, 39780 Lüleburgaz / Kirklareli (TR); ADIYAMAN, Mustafa, 39780 Lüleburgaz / Kirklareli (TR)
(74) Representative: Jirotkova, Ivana
(86) International application number: PCT/EP2013/003805
(87) International publication number: WO 2014/101986

(56) References cited:
- WO-A1-2008/101943
- WO-A1-2009/091663
- WO-A2-2007/078726

## Description

### Field of the Invention

The present invention relates to a novel dry granulation method for preparing metformin compositions as well as its combinations with DPP-IV inhibitors. The invention further relates to a novel composition of metformin per se and combinations thereof with DPP-IV inhibitors.

### Background of the Invention

Diabetes mellitus (diabetes) is a disease characterized by high blood glucose levels that result from defects in the body's ability to produce and/or use insulin. Diabetes mellitus occurs when the level of glucose in the blood becomes higher than normal. Main types of diabetes are type I diabetes and type II diabetes and also gestational diabetes. Type II diabetes is the most common form of diabetes, affecting 85-90% of all people suffering of this disorder. In type II diabetes mellitus the disease typically develops in later life. Insulin secretion may appear normal or even excessive (and type II patients are thus less prone to ketosis) but it is insufficient to compensate for insulin resistance. Obesity is present in the majority of type II patients; non-obese patients tend to have low insulin secretory capacity (although not as low as in type I diabetes) rather than appreciable insulin resistance. It is closely associated with cardiovascular disease.

Metformin is a well-known oral antidiabetic drug in the biguanide class which is described in the U.S. Patent No. 3,174,901. Metformin is currently marketed in 500, 850 and 1000 mg doses under the trademark Glucophage® for the treatment of Type II diabetes.

Metformin is a 1,1-dimethylbiguanide having the following structural formula:

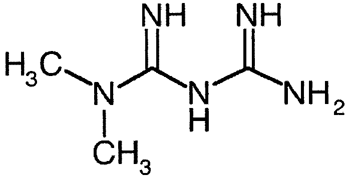

Vildagliptin is an inhibitor of the enzyme dipeptidylpeptidase-4, an enzyme responsible, among other roles, for the degradation of the incretion hormone glucagon-like peptide-1 (GLP-1; insulinotropin), which plays a role in regulating insulin secretion. Vildagliptin is used in the treatment of type II diabetes mellitus; it may be added to metformin, a sulfonylurea, or a thiazolidinedione, when monotherapy with these is insufficient.

Vildagliptin having the systematic name (S)-{[(3-Hydroxyadamantan-1-yl)amino]acetyl) pyrrolidine-2-carbonitrile and the following structural formula is marketed in a 50 mg dose under the trademark Galvus®.

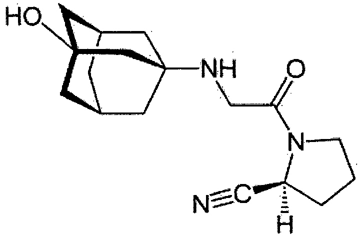

Combining two or more anti-diabetic agents into a single tablet provides a potential means of delivering combination therapy without adding to the complexity of patients' daily regimens, as described in EP 02402342. A combination of vildagliptin and metformin is marketed under the trade name Eucreas®. Eucreas combines two antihyperglycemic agents with complimentary mechanisms of action to improve glycemic control in patients with type II diabetes: vildagliptin, a member of the islet enhancer class, and metformin hydrochloride, a member of the biguanide class.

Flow properties and compressibility of metformin is known to be very inconvenient for tableting process. As it is highly crystalline and has poor compaction properties, it is difficult to form tablets by direct compression or dry granulation. In general, wet granulation process is used for pharmaceutical ingredients which have such properties. Metformin further, is a high dose drug that is hard to compress directly into tablets. In this instance, the problems with overweighed tablets arise upon dry granulation processes since the resulting powder requires excessive amount of excipients, and especially binders, to obtain properly compressed tablets.

US 2003/104049-A1 discloses a dry-mix process for preparing Metformin formulations with methocel 15cps, microcrystalline cellulose and magnesium stearate in different proportions (Examples 1-6). It is however noted that amount of the excipients needed in the formulation is rather high, and also use of lubricants such as magnesium stearate is very limited due to their adverse effects in terms of lower hardness and friability (par. 24). The applicants of this application report that as the amount of magnesium stearate is reduced in the examples the hardness and friability are dramatically improved. However, those skilled in the art would appreciate that decreasing the amount of the lubricant shall cause unavoidable problems such as low flowability and sticking problems in the punches. Therefore, there still exists the need of metformin formulations which have good friability and hardness without suffering of sticking and poor flowability.

Thus, one of the objects of the present invention is to provide metformin formulations that do not suffer of the problems set out above.

Another objective of the present invention is to develop formulations that make the dry granulation process more consistent and therefore feasible for industrial production, while maintaining pharmaceutical equivalence to the reference pharmaceutical form.

Moreover, dipeptidyl peptidase-4 (DPP-4) inhibitor class of drugs, especially Vildagliptin are hygroscopic, practically not compactible and having stability problems. Since vildagliptin is a moisture sensitive molecule, can lead to product stability issues. To be able to overcome this problem the inventors have developed a dry granulation process and a specific composition in order to obtain a free-flowing, compactable composition which leads compressing of tablets having good properties e.g. good hardness, friability, and with improved stability of Vildagliptin.

WO 2007/078726-A2 discloses dry or wet processing methods for preparing metformin and DPP-4 inhibitor combinations, although all formulations described in the specific examples are stated as being prepared via wet granulation method. This document is silent with respect to problems of compressibility and stability neither does it disclose a specific formulation that is prepared via direct or dry granulation. Also tablet weight and friability are not taken as a concern in dry granulation method.

WO 2007/041053-A2 discloses preparation of DPP-4 inhibitor/metformin combinations where it is stated that wet granulation can be preferred rather than the dry granulation because of the advantages it confers such as compressibility with better wetting properties, reducing dissolution and disintegration problems, and providing improved content uniformity. It is further noted that metformin is typically produced by a wet granulation process with a high drug load and is known to be very difficult to process with conventional techniques such as roller compaction and direct compression because of the poor compaction properties. To solve the problems set out above, suitable excipients such as binders are given a particular weight whereas diluents are seen as entirely optional. Wet and melt granulations along with binders are proposed as the preferable methods, and it is also recited that water granulated metformin exhibits a vildagliptin degradation of 6.6% according to Example 2. No specific examples are given for metformin formulations prepared via dry granulation techniques.

WO 2011/032912-A1 discloses immediate release formulation of Metformin and Vildagliptin or Sitagliptin combinations, as well as methods for manufacturing such formulations. Direct compression and melt granulation methods where Metformin hydrochloride is used as a mixture with 0.5% Aerosil® are applied. It is proposed that the active substances are processed into the pharmaceutical composition together with more than 10 wt. % of lubricant, wherein the lubricant is polyethylene glycol or a mixture of polyethylene glycol with one or plurality of other lubricants.

According to the prior art, DPP-IV inhibitor particles may also be added to outer phase of the Metformin granules that are blended and tableted. However, for a DPP-IV inhibitor which has %3-4 load in the final composition, blend uniformity risk must then be taken into consideration. It is known that, wet/melt granulation of Metformin and DPP-IV inhibitor together, leads degradation issues of DPP-IV inhibitor as mentioned in WO 2007/041053-A2.

Thus, it is a further object of the present invention to eliminate the risk of DPP-IV inhibitor blend uniformity at the final composition.

To this end, elimination of the drawbacks associated with classical wet/melt/dry granulation methods in preparation of compressible tablet powders of Metformin and DPP-4 inhibitors, and providing the improved tablets *per se* will be a considerable development in the formulation science.

It is therefore, a further object of the present invention to eliminate complicated and costly steps of wet/melt granulation in production of compressible Metformin - DPP-4 inhibitor combinations.

More specifically, it is an object of the present invention to provide a straightforward method for producing such combinations by modifying the excipients and thereby obtaining better compressed tablets without suffering of sticking and flowability problems.

A further object of the present invention is to eliminate adverse effects of conventional methods on Metformin - DPP-4 inhibitor combinations to ensure an improved stability.

Objects of the present invention are achieved through using specific diluents, i.e. a combination of microcrystalline cellulose and dibasic calcium phosphate anhydrous in properly arranged amounts.

### Detailed Description of the Invention

The term "metformin" as employed herein refers to metformin or a pharmaceutically acceptable salt thereof. In the present context, "a DPP-IV inhibitor" refers also to active metabolites and prodrugs of DPP-IV inhibitors. A "metabolite" is an active derivative of a DPP-IV inhibitor which is produced when the DPP-IV inhibitor is metabolized. A "prodrug" is a compound that is either metabolized to a DPP-IV inhibitor or is metabolized to the same metabolite(s) as a DPP-IV inhibitor. In preferred embodiments, said DPP-IV inhibitor includes sitagliptin, vildagliptin, or saxagliptin. Most preferably, the DPP-IV inhibitor is vildagliptin.

As noted in the background art, metformin and DPP-IV inhibitors exhibit certain disadvantages rendering them difficult to process via conventional techniques. Flowability, friability, compressibility and stability are the major ones that need to be dealt with in developing novel methods and formulations. To be more specific, metformin suffers of poor compaction and flowability whereas both of metformin and DPP-IV inhibitors suffer of poor or moderately poor stability if wet granulation technique is used, because of vildagliptin's moisture sensivity. Segregation is a further problem of DPP-4 inhibitors that is generally caused by the crystal structure of these compounds.

Wet granulation methods as proposed in prior art for elimination of compressibility problems related to metformin are still found to be not sufficient to overcome stability and potential blend uniformity problems of the drug combinations. On the other hand, it is known that conventional methods of dry granulation fail to provide a satisfactory solution to poor compaction properties of metformin and this causes the tablets to be prepared as overweighed because of the requirement to use excessive amount of excipients.

Having regard to the objectives of the present invention, the inventors performed extensive research on novel formulations of Metformin/DPP-4 inhibitor combinations that are easily compressible and sufficiently stable, and surprisingly found that certain diluents, i.e. combinations of Microcrystalline Cellulose (MCC) and Dibasic Calcium Phosphate Anhydrous (DCPA), are advantageous for elimination of the drawbacks noted, for instance in dry granulation methods. MCC and DCPA are noted to be useful for obtaining better compaction and flowability of metformin when they are used as a combination. The inventors further note that the aforesaid beneficial effects are much more observable when metformin:diluent (MCC+DCPA) ratio is 2.5-25 by weight even though the said diluent having much lower weight ratios is noted to be still effective in terms of successfully compressed tablets with good friability and low sticking problems. In this instance, lower amounts of MCC+DCPA such as a Metformin:Diluent weight ratio of 15-25 are effective as well, and such lower amounts of diluents considerably decreases the tablet load. Therefore, negative impacts of lubricants (i.e. magnesium stearate) as stated for instance in US 2003/0104049, causing high friability and hardness are currently eliminated. Besides that, stability problems associated with wet methods or excessive amounts of excipients reactive to DPP-4 inhibitors can also be eliminated.

Thus, according to first aspect of the present invention, there is provided a method for producing tablet compositions of Metformin comprising dry granulation of the active substance with MCC+DCPA. The composition preferably further comprises a DPP-4 inhibitor. In further embodiments the ratio of Metformin:MCC+DCPA is 2.5-25 by weight.

According to a further aspect, there is provided a composition of Metformin comprising a combination of MCC and DCPA as diluents. Accordingly, the ratio of Metformin:(MCC + DCPA) can be 2.5-25 by weight.

As noted above, DPP-IV inhibitor is preferably vildagliptin. The method of the present invention aiming at preparing advantageous tablet compositions may include also further diluents, binders, disintegrants, lubricants and glidants.

Examples of those further diluents include mannitol, lactose sorbitol, dibasic calcium phosphate hydrous and powdered cellulose. The total amount of the diluents added to the granulation procedure is 2-25%, more preferably 10-20% based on the total weight of the formulation.

To be able to produce a tablet which has a suitable size for patients' use i.e. ease to swallow; diluent selection was a critical point. For 1000mg Metformin and 50mg Vildagliptin tablets, the target was 1200mg - 1600mg tablet weight which is corresponding to %83.3 - %62.5 Metformin load. At the first trial, inventors evaluated numerous excipients such as lactose as a diluent, but to achieve sufficient granule formation by dry granulation, large amount of lactose is consumed, hence larger tablet sizes are obtained. It is found that, Microcrystalline Cellulose in combination with Dibasic Calcium Phosphate Anhydrous gave promising results with regards to good compaction properties with lower percentage in the composition, and tablets between the margins of targeted weight with sufficient hardness and friability and without sticking to the punches were obtained. Avicel PH101 and Avicel DG are used for several trials and re-compactibility of DG grade was considerably better than PH101 grade, such as higher hardness values and desired dissolution profiles are obtained for final tablets with Avicel DG. Avicel DG is known to be a combination of 75% Microcrystalline Cellulose and 25% Dibasic Calcium Phosphate Anhydrous and noted to be useful for carrying out the objectives of the present invention both for metformin and its combination with DPP-4 inhibitors.

The novel method of the present invention may also involve one or more binding agents to be used in the dry granulation procedure. Examples of binding agents include hydroxypropylcellulose (HPC), hydroxypropylmethyl cellulose (HMPC), hydroxyethyl cellulose, starch 1500, polyvinylpyrrolidone (povidone), and copovidone. A preferred binding agent is hydroxypropylcellulose (HPC). Binders can be present at an amount of 1-10%, more preferably 3-5% by weight of the overall composition.

According to a further embodiment, the method of the present invention may also involve a disintegrant. The disintegrant maybe one or more of modified starches, modified cellulose polymers, or polycarboxylic acids, such as crosslinking hydroxymethylcellulose sodium, starch sodium glycollate, polacrillin potassium, croscarmellose sodium and hydroxymethylcellulose calcium (HMC-Ca). The disintegrant is preferably croscarmellose sodium, and can be present at an amount of 0.1-5%, more preferably 0.5-2% by weight of the overall composition.

The method of the present invention may also involve one or more lubricants and/or glidants that are selected from the group of magnesium stearate, calcium stearate, stearic acid, sodium stearyl fumarate, hydrogenated castor oil, talc, fumaric acid, starches and mixture thereof. A preferred lubricant is magnesium stearate or sodium stearyl fumarate or a mixture thereof. Examples of glidants include colloidal silicon dioxide, calcium phosphate, magnesium silicate, and talc. Lubricants and glidants can be present at an amount of 0.1-5%, more preferably 0.5-2% by weight of the overall composition. As mentioned above, the compositions of the present invention do not bear the drawbacks of the lubricants as seen in the conventional compositions.

The method of the invention may further involve surfactants or wetting agents. The surfactant may be anionic, cationic, or neutral. Anionic surfactants include sodium lauryl sulfate, sodium dodecanesulfonate, sodium oleyl sulfate, and sodium laurate mixed with stearates and talc. Cationic surfactants include benzalkonium chlorides and alkyltrimethylammonium bromides. Neutral surfactants include glyceryl monooleate, polyoxyethylene dehydrated sorbitan fatty acid ester, polyvinyl alcohol, and dehydrated sorbitan ester.

In preferred embodiments of the present invention Metformin may be involved in the dry granulation procedure in the form of its HCl salt, and is present at an amount of 60-98%, more preferably 70-80% by weight of the overall composition. DPP-4 inhibitor is also present at an amount of 1-6%, more preferably 4-5% by weight of the overall composition.

The novel method of the present invention ensures good compressibility of the ingredients in the combinations even in highly loaded drugs containing excessive amount of active material as specified above. In preferred embodiments the diluents include 75% MCC + 25% DCPA, such as Avicel DG, for more effectively solving the stability problems of the composition and making easier the processing of the same.

The invention will be further defined with reference to specific examples herein below.

### Example I

### Preparation of oral tablets comprising Metformin HCl and Vildagliptin

### Preparation of Powder Mixture

Metformin HCl was sieved through a 850 µm sieve, also Vildagliptin, Avicel DG and HPC were sieved through a 500 µm sieve. Colloidal anhydrous silica was then weighed onto other ingredients and blended. Powder mixture was then sieved through 850 µm sieve and blended.

### Slugging

Powder mixture is compressed into 900 mg slug tablets with 50N hardness. The slug tablets are sieved through 1.0 mm frewitt sieve. Croscarmellose Sodium is added and blended, and Magnesium stearate is added to the granules whereas final mixture was continued to be blended.

### Tableting

The final mixture was then compressed into tablets in a rotary tableting machine. While the tablet hardness was around 200 N, the disintegration time was around 16 minutes. The tablets obtained with the procedure above were having the following composition.

### Film Coating

%15 dispersion of coating material is prepared and cores are coated until an approximately %2-3 weight gain is obtained.

**Table I. Formulation-1 according to Example I**

| | **INGREDIENTS** | **UNIT FORMULA (mg / tabl.)** |
|---|---|---|
| Active ingredient - 1 | Metformin HCl | 1000.00 |
| Active ingredient - 2 | Vildagliptin | 50.00 |
| Diluent | Microcrystalline Cellulose+DCPA (Avicel DG) | 187.50 |
| Binder | Hydroxy Propyl Cellulose (Klucel EXF) | 68.00 |
| Disintegrant | Croscarmellose Sodium | 13.50 |
| Glidant | Colloidal Silicon dioxide (Aerosil) | 21.00 |
| Lubricant | Magnesium stearate | 10.00 |
| TOTAL | | 1350.00 |
| Coating material | Hypromellose, Titanium dioxide, Yellow iron oxide, Polyethylene glycol, Talc | 32.40 |

**Table II. Formulation-2 according to Example I**

| | **INGREDIENTS** | **UNIT FORMULA (mg / tabl.)** |
|---|---|---|
| Active ingredient - 1 | Metformin HCl | 1000.00 |
| Active ingredient - 2 | Vildagliptin | 50.00 |
| Diluent | Microcrystalline Cellulose+DCPA (Avicel DG) | 60.00 |
| Binder | Hydroxy Propyl Cellulose (Klucel EXF) | 60.00 |
| Disintegrant | Croscarmellose Sodium | 7.00 |
| Glidant | Colloidal Silicon dioxide (Aerosil) | 21.00 |
| Lubricant | Magnesium stearate | 10.00 |
| TOTAL | | 1208.00 |
| Coating material | Hypromellose, Titanium dioxide, Yellow iron oxide, Polyethylene glycol, Talc | 28.70 |

### Example II

### Preparation of oral tablets comprising Metformin HCl and Vildagliptin

### Preparation of Powder Mixture

Metformin HCl was sieved through a 850 µm sieve, also Vildagliptin, Avicel DG and HPC were sieved through a 500 µm sieve. Colloidal anhydrous silica, Croscarmellose Sodium were then weighed onto other ingredients and blended. Powder mixture was then sieved through 850 µm sieve and blended.

### Compaction

Powder mixture was roller compacted according to 100 bar hydraulics, 40 rpm screw feeder, 7 rpm roller unit, 25 rpm fine granulator, 1.0 mm roller gap using 1.0 mm sieve.

### Tableting

The final mixture was then compressed into tablets in a rotary tableting machine. While the tablet hardness was around 200 N, the disintegration time was around 16 minutes. The tablets obtained with the procedure above were having the following composition.

### Film Coating

%15 dispersion of coating material is prepared and cores are coated until an approximately %2-3 weight gain is obtained.

**Table III. Formulation-1 according to Example II**

| | **INGREDIENTS** | **UNIT FORMULA (mg / tabl.)** |
|---|---|---|
| Active ingredient - 1 | Metformin HCl | 1000.00 |
| Active ingredient - 2 | Vildagliptin | 50.00 |
| Diluent | Microcrystalline Cellulose+DCPA (Avicel DG) | 187.50 |
| Binder | Hydroxy Propyl Cellulose (Klucel EXF) | 68.00 |
| Disintegrant | Croscarmellose Sodium | 13.50 |
| Glidant | Colloidal Silicon dioxide (Aerosil) | 21.00 |
| Lubricant | Magnesium stearate | 10.00 |
| TOTAL | | 1350.00 |
| Coating material | Hypromellose, Titanium dioxide, Yellow iron oxide, Polyethylene glycol, Talc | 32.40 |

**Table IV. Formulation-2 according to Example II**

| | **INGREDIENTS** | **UNIT FORMULA (mg / tabl.)** |
|---|---|---|
| Active ingredient - 1 | Metformin HCl | 1000.00 |
| Active ingredient - 2 | Vildagliptin | 50.00 |
| Diluent | Microcrystalline Cellulose+DCPA (Avicel DG) | 60.00 |
| Binder | Hydroxy Propyl Cellulose (Klucel EXF) | 60.00 |
| Disintegrant | Croscarmellose Sodium | 7.00 |
| Glidant | Colloidal Silicon dioxide (Aerosil) | 21.00 |
| Lubricant | Magnesium stearate | 10.00 |
| TOTAL | | 1208.00 |
| Coating material | Hypromellose, Titanium dioxide, Yellow iron oxide, Polyethylene glycol, Talc | 28.70 |

**Table V. Formulation-3 according to Example II**

| | **INGREDIENTS** | **UNIT FORMULA (mg / tabl.)** |
|---|---|---|
| Active ingredient - 1 | Metformin HCl | 1000.00 |
| Active ingredient - 2 | Vildagliptin | 50.00 |
| Diluent-1 | Microcrystalline Cellulose+DCPA (Avicel DG) | 60.00 |
| Diluent-2 | Lactose | 60.00 |
| Binder | Hydroxy Propyl Cellulose (Klucel EXF) | 68.00 |
| Disintegrant | Croscarmellose Sodium | 13.50 |
| Glidant | Colloidal Silicon dioxide (Aerosil) | 21.00 |
| Lubricant | Magnesium stearate | 10.00 |
| TOTAL | | 1282.50 |
| Coating material | Hypromellose, Titanium dioxide, Yellow iron oxide, Polyethylene glycol, Talc | 30.80 |

### Example III (Comparative Tests)

Formulations with different amounts of excipients were prepared for assessment of the effect of each diluent on flowability, friability, hardness and compressibility of the resulting tablets. Compositions of the formulations are set out in Table VI below.

As noted, Formulas 1, 4 and 5 were prepared with different amounts of Avicel DG while Formulas 2 and 3 were prepared with lactose and DCPA respectively.

Flowability and friability of the compressed tablets obtained with the formulations are determined according to the Hausner ratio which corresponds to the ratio of Tapped density to Bulk density. Table VII given below shows scaling of flowability depending on the value of the Hausner ratio.

**Table VI. Formulas 1-5 with different diluents**

| Function | Name of Substance | Formula - 1 | Formula - 2 | Formula - 3 | Formula - 4 | Formula-5 |
|---|---|---|---|---|---|---|
| Active | Metformin HCl | 1000.0 | 1000.0 | 1000.0 | 1000.0 | 1000.0 |
| Active | Vildagliptin | 50.0 | 50.0 | 50.0 | 50.0 | 50.0 |
| Diluent | DCPA | - | - | 187.5 | - | - |
| | Avicel DG | 60.0 | - | - | 60.0 | 187.5 |
| | Lactose | - | 187.5 | - | 60.0 | - |
| Binder | Klucel EXF | 60.0 | 68.0 | 68.0 | 68.0 | 68.0 |
| Disintegrant | Croscarmellose Na | 7.0 | 13.5 | 13.5 | 13.5 | 13.5 |
| Glidant | Aerosil | 21.0 | 21.0 | 21.0 | 21.0 | 21.0 |
| Lubricant | Magnesium Stearate | 10.0 | 10.0 | 10.0 | 10.0 | 10.0 |
| TOTAL | | 1208.0 | 1350.0 | 1350.0 | 1282.5 | 1350.0 |
| Bulk density | | 0.64 | 0.67 | 0.68 | 0.65 | 0.78 |
| Tapped density | | 0.84 | 0.84 | 0.87 | 0.82 | 0.92 |
| Hausner Ratio | | 1.31 | 1.25 | 1.28 | 1.26 | 1.18 |
| Angle of response | | 40.2 | 40.7 | 43.4 | 40.7 | 40.5 |

**Table VII. Scale of Flowability**

| **Compressibility Index** | **Flow Character** | **Hausner Ratio** |
|---|---|---|
| ≤10 | Excellent | 1.00-1.11 |
| 11-15 | Good | 1.12.1.18 |
| 16-20 | Fair | 1.19-1.25 |
| 21-25 | Passable | 1.26-1.34 |
| 26-31 | Poor | 1.35.1.45 |
| 32-37 | Very poor | 1.46-1.59 |
| >38 | Very, very poor | >1.60 |

According to Table VI, best results were obtained with Formula-5 which contains relatively a higher amount of Avicel DG. Notably, Formula-2 and Formula-3 having the same amount of lactose and dibasic calcium phosphate anhydrous respectively instead of Avicel DG had fairly lower flow characteristics as compared to Formula-5.

It was also surprisingly noted that Avicel DG improves friability and compressibility of the formulations. Although lower amounts of Avicel DG causes lower flowability, the inventors report that friability and tableting features of Formulas 1 and 4 were still acceptable despite the fact that magnesium stearate, which is known to be detrimental to friability, is involved in all formulations.

## Claims

1. A method for producing tablet compositions of Metformin comprising dry granulation of the active substance with a diluent which is a mixture of Microcrystalline Cellulose and Dibasic Calcium Phosphate Anhydrous.

2. A method according to claim 1 wherein the ratio of Metformin:Diluent is 2.5-25 by weight.

3. A method according to claim 1 wherein the method further comprises addition of a DPP-4 inhibitor to the composition to produce a combination of Metformin and said DPP-4 inhibitor.

4. A method according to claim 1 wherein said DPP-4 inhibitor is selected from the group consisting of sitagliptin, vildagliptin, and saxagliptin.

5. A method according to claim 4 wherein the DPP-4 inhibitor is vildagliptin.

6. A method according to claim 1 wherein the mixture of Microcrystalline Cellulose and Dibasic Calcium Phosphate Anhydrous is Avicel DG.

7. A tablet composition comprising dry granulated Metformin granules and a diluent which is a mixture of Microcrystalline Cellulose and Dibasic Calcium Phosphate Anhydrous.

8. A composition according to claim 7 wherein the ratio of Metformin:Diluent is 2.5-25 by weight.

9. A composition according to claim 7 further comprising a DPP-4 inhibitor.

10. A composition according to claim 9 wherein said DPP-4 inhibitor is selected from the group consisting of sitagliptin, vildagliptin, and saxagliptin.

11. A composition according to claim 10 wherein the DPP-4 inhibitor is vildagliptin.

12. A composition according to claim 7 wherein the mixture of Microcrystalline Cellulose and Dibasic Calcium Phosphate Anhydrous is Avicel DG.

13. A composition according to claim 7 wherein the composition further comprises one or more excipients selected from the group of further diluents, binders, disintegrants, lubricants and glidants.

14. A composition according to claim 13 wherein the further diluent is selected from the group consisting of mannitol, lactose, sorbitol, dibasic calcium phosphate hydrous and powdered cellulose.

15. A composition according to claim 7 or 13 wherein total amount of the diluents added to the composition is 2-25% by weight of the overall composition.

16. A composition according to claim 13 wherein the binder is selected from the group of hydroxypropylcellulose, hydroxypropylmethyl cellulose, hydroxyethyl cellulose, starch, polyvinylpyrrolidone, and copovidone.

17. A composition according to claim 13 wherein the disintegrant is selected from the group of modified starches, modified cellulose polymers, crosslinking hydroxymethylcellulose sodium, starch sodium glycollate, polacrillin potassium, croscarmellose sodium and hydroxymethylcellulose calcium.

18. A composition according to claim 13 wherein the lubricant and glidant is selected from the group of magnesium stearate, calcium stearate, stearic acid, sodium stearyl fumarate, hydrogenated castor oil, talc, colloidal silicon dioxide, fumaric acid, starches and mixtures thereof.

19. A composition according to claim 7 wherein amount of metformin is 60-98% by weight of the overall composition.

20. A composition according to claim 9 wherein amount of the DPP-4 inhibitor is 1-6% by weight of the overall composition.

21. A composition according to any of the preceding claims wherein mixture of the composition comprises the following ingredients:
| **Ingredient** | **Amount (%) by weight of the overall composition** |
|---|---|
| Metformin HCl | 70-80% |
| Vildagliptin | 4-5% |
| Diluent | 10-20% |
| Binder | 3-5% |
| Disintegrant | 0.5-2% |
| Lubricant | 0.5-2% |
| Glidant | 0.5-2% |

22. A composition according to any of the preceding claims wherein said composition comprises active ingredients corresponding to one of the dosage amounts of 500mg, 850mg,1000mg Metformin and of 25mg, 50mg and 100mg Vildagliptin free bases.

## Patentansprüche

1. Verfahren zur Herstellung von Tablettenzusammensetzungen von Metformin, umfassend die Trockengranulation des Wirkstoffs mit einem Verdünnungsmittel, das ein Gemisch aus mikrokristalliner Cellulose und wasserfreiem zweibasigem Calciumphosphat ist.

2. Verfahren nach Anspruch 1, wobei das Verhältnis von Metformin:Verdünnungsmittel 2,5-25, bezogen auf das Gewicht, beträgt.

3. Verfahren nach Anspruch 1, wobei das Verfahren ferner das Hinzufügen eines DPP-4-Inhibitors zu der Zusammensetzung umfasst, um eine Kombination von Metformin und dem DPP-4-Inhibitor zu erhalten.

4. Verfahren nach Anspruch 1, wobei der DPP-4-Inhibitor aus der Gruppe bestehend aus Sitagliptin, Vildagliptin und Saxagliptin ausgewählt ist.

5. Verfahren nach Anspruch 4, wobei der DPP-4-Inhibitor Vildagliptin ist.

6. Verfahren nach Anspruch 1, wobei das Gemisch aus mikrokristalliner Cellulose und wasserfreiem zweibasigem Calciumphosphat Avicel DG ist.

7. Tablettenzusammensetzung, umfassend trockengranuliertes Metformingranulat und ein Verdünnungsmittel, das ein Gemisch aus mikrokristalliner Cellulose und wasserfreiem zweibasigem Calciumphosphat ist.

8. Zusammensetzung nach Anspruch 7, wobei das Verhältnis von Metformin:Verdünnungsmittel 2,5-25, bezogen auf das Gewicht, beträgt.

9. Zusammensetzung nach Anspruch 7, ferner umfassend einen DPP-4-Inhibitor.

10. Zusammensetzung nach Anspruch 9, wobei der DPP-4-Inhibitor aus der Gruppe ausgewählt ist, die aus Sitagliptin, Vildagliptin und Saxagliptin besteht.

11. Zusammensetzung nach Anspruch 10, wobei der DPP-4-Inhibitor Vildagliptin ist.

12. Zusammensetzung nach Anspruch 7, wobei das Gemisch aus mikrokristalliner Cellulose und wasserfreiem zweibasigem Calciumphosphat Avicel DG ist.

13. Zusammensetzung nach Anspruch 7, wobei die Zusammensetzung weiterhin einen oder mehrere Hilfsstoffe, ausgewählt aus der Gruppe weitere Verdünnungsmittel, Bindemittel, Sprengmittel, Gleitmittel und Fließregulierungsmittel, umfasst.

14. Zusammensetzung nach Anspruch 13, wobei das weitere Verdünnungsmittel aus der Gruppe ausgewählt ist, die aus Mannit, Lactose, Sorbit, wasserhaltigem zweibasigem Calciumphosphat und pulverisierter Cellulose besteht.

15. Zusammensetzung nach Anspruch 7 oder 13, wobei die Gesamtmenge der zu der Zusammensetzung gegebenen Verdünnungsmittel 2-25 Gew.-% der Gesamtzusammensetzung beträgt.

16. Zusammensetzung nach Anspruch 13, wobei das Bindemittel aus der Gruppe Hydroxypropylcellulose, Hydroxypropylmethylcellulose, Hydroxyethylcellulose, Stärke, Polyvinylpyrrolidon und Copovidon ausgewählt ist.

17. Zusammensetzung nach Anspruch 13, wobei das Sprengmittel aus der Gruppe modifizierte Stärken, modifizierte Cellulosepolymere, vernetzte Hydroxymethylcellulose-Natrium, Stärke-Natriumglycolat, Polacrilin-Kalium, Croscarmellose-Natrium und Hydroxymethylcellulose-Calcium ausgewählt ist.

18. Zusammensetzung nach Anspruch 13, wobei das Gleitmittel und das Fließregulierungsmittel aus der Gruppe Magnesiumstearat, Calciumstearat, Stearinsäure, Natriumstearylfumarat, hydriertes Rizinusöl, Talkum, kolloidales Siliciumdioxid, Ameisensäure, Stärken und Mischungen davon ausgewählt sind.

19. Zusammensetzung nach Anspruch 7, wobei die Menge an Metformin 60-98 Gew.-% der Gesamtzusammensetzung ausmacht.

20. Zusammensetzung nach Anspruch 9, worin die Menge des DPP-4-Inhibitors 1-6 Gew.-% der Gesamtzusammensetzung ausmacht.

21. Zusammensetzung nach einem der vorhergehenden Ansprüche, wobei das Gemisch der Zusammensetzung die folgenden Bestandteile umfasst:
| Bestandteil | Menge (%), bezogen auf das Gewicht der Gesamtzusammensetzung |
|---|---|
| Metformin HCl | 70-80 % |
| Vildagliptin | 4-5 % |
| Verdünnungsmittel | 10-20 % |
| Bindemittel | 3-5 % |
| Sprengmittel | 0,5-2 % |
| Gleitmittel | 0,5-2 % |
| Fließregulierungsmittel | 0,5-2 % |

22. Zusammensetzung nach einem der vorhergehenden Ansprüche, wobei die Zusammensetzung Wirkstoffe entsprechend einer der Dosierungsmengen 500 mg, 850 mg, 1000 mg Metformin und 25 mg, 50 mg und 100 mg Vildagliptin (freie Basen) umfasst.

## Revendications

1. Procédé de production de compositions de comprimés de metformine, comprenant la granulation sèche de la substance active avec un diluant qui est un mélange de cellulose microcristalline et de phosphate de calcium dibasique anhydre.

2. Procédé selon la revendication 1, dans lequel le rapport metformine/diluant est de 2,5 à 25 en poids.

3. Procédé selon la revendication 1, dans lequel le procédé comprend en outre l'addition d'un inhibiteur de DPP-4 à la composition pour produire une combinaison de metformine et dudit inhibiteur de la DPP-4.

4. Procédé selon la revendication 1, dans lequel ledit inhibiteur de la DPP-4 est choisi dans le groupe constitué par la sitagliptine, la vildagliptine et la saxagliptine.

5. Procédé selon la revendication 4, dans lequel l'inhibiteur de la DPP-4 est la vildagliptine.

6. Procédé selon la revendication 1, dans lequel le mélange de cellulose microcristalline et de phosphate de calcium dibasique anhydre est Avicel DG.

7. Composition de comprimé comprenant des granulés de metformine granulés à sec et un diluant qui est un mélange de cellulose microcristalline et de phosphate de calcium dibasique anhydre.

8. Composition selon la revendication 7, dans laquelle le rapport de metformine/diluant est de 2,5 à 25 en poids.

9. Composition selon la revendication 7, comprenant en outre un inhibiteur de la DPP-4.

10. Composition selon la revendication 9, dans laquelle ledit inhibiteur de la DPP-4 est choisi dans le groupe constitué par la sitagliptine, la vildagliptine et la saxagliptine.

11. Composition selon la revendication 10, dans laquelle l'inhibiteur de la DPP-4 est la vildagliptine.

12. Composition selon la revendication 7, dans laquelle le mélange de cellulose microcristalline et de phosphate de calcium dibasique anhydre est Avicel DG.

13. Composition selon la revendication 7, dans laquelle la composition comprend en outre un ou plusieurs excipients choisis dans le groupe des diluants, liants, agents de désintégration, lubrifiants et agents de glissement.

14. Composition selon la revendication 13, dans laquelle l'autre solvant est choisi dans le groupe constitué par le mannitol, le lactose, le sorbitol, le phosphate dibasique de calcium hydraté et la cellulose en poudre.

15. Composition selon la revendication 7 ou 13, dans laquelle la quantité totale des diluants ajoutés à la composition est de 2-25% en poids de la composition totale.

16. Composition selon la revendication 13, dans laquelle le liant est choisi dans le groupe constitué par l'hydroxypropylcellulose, l'hydroxypropylméthylcellulose, l'hydroxyéthylcellulose, l'amidon, la polyvinylpyrrolidone et la copovidone.

17. Composition selon la revendication 13, dans laquelle l'agent de désintégration est choisi dans le groupe des amidons modifiés, des polymères de cellulose modifiés, l'hydroxyméthylcellulose de sodium réticulant, le glycolate de sodium et d'amidon, la polacrilline de potassium, la croscarmellose sodique et l'hydroxyméthylcellulose de calcium.

18. Composition selon la revendication 13, dans laquelle le lubrifiant et l'agent de glissement sont choisis dans le groupe constitué par le stéarate de magnésium, le stéarate de calcium, l'acide stéarique, le stéarylfumarate de sodium, l'huile de ricin hydrogénée, le talc, le dioxyde de silicium colloïdal, l'acide fumarique, les amidons et leurs mélanges.

19. Composition selon la revendication 7, dans laquelle la quantité de metformine est de 60 à 98% en poids de la composition totale.

20. Composition selon la revendication 9, dans laquelle la quantité de l'inhibiteur de la DPP-4 est 1-6% en poids de la composition totale.

21. Composition selon l'une quelconque des revendications précédentes, dans laquelle le mélange de la composition comprend les ingrédients suivants:
| **Ingrédient** | **Quantité (%) en poids de la composition totale** |
|---|---|
| Metformine HCl | 70-80% |
| Vildagliptine | 4-5% |
| Diluant | 10-20% |
| Liant | 3-5% |
| Agent de désintégration | 0.5-2% |
| Lubrifiant | 0.5-2% |
| Agent de glissement | 0.5-2% |

22. Composition selon l'une quelconque des revendications précédentes, dans laquelle ladite composition comprend des ingrédients actifs correspondant à l'un des dosages de 500mg, 850mg, 1000mg de metformine et de 25mg, 50mg et 100mg de vildagliptine sous forme de base libre.
